Europäisches Patentamt

European Patent Office

Office européen des brevets

(11) Veröffentlichungsnummer: **0 330 863 B1**

(12) **EUROPÄISCHE PATENTSCHRIFT**

(45) Veröffentlichungstag der Patentschrift: **12.05.93**

(51) Int. Cl.⁵: **A61K 6/04**

(21) Anmeldenummer: **89102050.5**

(22) Anmeldetag: **07.02.89**

(54) **Verwendung von Palladium−Silber−Legierungen zur Herstellung von Zahnersatz.**

(30) Priorität: **27.02.88 DE 3806343**

(43) Veröffentlichungstag der Anmeldung:
**06.09.89 Patentblatt 89/36**

(45) Bekanntmachung des Hinweises auf die
Patenterteilung:
**12.05.93 Patentblatt 93/19**

(84) Benannte Vertragsstaaten:
**AT BE CH DE ES FR GB GR IT LI LU NL SE**

(56) Entgegenhaltungen:
**DE−B− 2 523 971**
**DE−C− 3 438 288**
**NL−A− 8 001 820**
**US−A− 4 350 526**

(73) Patentinhaber: **Degussa Aktiengesellschaft**
**Weissfrauenstrasse 9**
**W−6000 Frankturt am Main 1(DE)**

(72) Erfinder: **Groll, Werner, Dr. Dipl.−Ing.**
**Gartenstrasse 5**
**W−8755 Alzenau−Hörstein(DE)**
Erfinder: **Schöck, Gernot, Dipl.−Ing.**
**Bahnhofstrasse 56**
**W−6454 Bruchköbel(DE)**
Erfinder: **Hathaway, Doris**
**Lahnstrasse 20**
**W−6450 Hanau 7(DE)**
Erfinder: **Stümke, Manfred, Dr. Dipl.−Phys.**
**Am Walsweg 21**
**W−7530 Pforzheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft die Verwendung von Palladium – Silberlegierungen zur Herstellung von festsit – zendem und herausnehmbaren, mit Dentalkeramik verblendbaren Zahnersatz.

Festsitzender und herausnehmbarer Zahnersatz wird vorwiegend aus korrosionsbeständigen, biokom – patiblen Edelmetallegierungen mit dem sogenannten Wachsausschmelzverfahren hergestellt, wobei das gegossene Objekt häufig mit Dentalkeramik verblendet wird, um ein dem natürlichen Zahn entsprechendes Aussehen zu erzielen. Hierfür müssen die Legierungen spezielle, auf die Dentalkeramik abgestimmte Eigenschaften besitzen, wie thermischen Ausdehnungskoeffizienten, Schmelzintervall oder Haftung.

Hochgoldhaltige Legierungen, wie sie z.B. in den deutschen Patentschriften 11 83 247 und 15 33 233 beschrieben sind, eignen sich für diesen Zweck besonders gut. Wegen des hohen, stark schwankenden Preises von Gold ist jedoch in letzter Zeit verstärkt nach preiswerteren Alternativen zu den hochgoldhaltigen Legierungen gesucht worden. Im Bereich der Edelmetalle bieten sich als Ersatz das Palladium wegen seines relativ günstigen Preises, seiner gegenüber Gold deutlich verringerten Dichte und seiner dem Gold vergleichbaren Korrosions – bzw. Mundbeständigkeit an.

Bei den bisher bekannten Palladiumbasislegierungen auf dem Dentalgebiet, kann man zwischen silberfreien und silberhaltigen Legierungen unterscheiden.

Silberfreie Palladiumbasislegierungen enthalten als Hauptlegierungselemente Kupfer, Zinn, Indium, Kobalt und Gallium. Typische silberfreie Palladiumbasislegierungen sind z.B. in den deutschen Patent – schriften 33 16 595, 33 04 183, 33 14 657 oder 35 22 523 beschrieben. Gegenüber den hochgoldhaltigen Legierungen reagieren diese empfindlicher auf Verarbeitungsfehler und sind schwer lötbar. Im schmelz – flüssigen Zustand nehmen sie erhebliche Mengen Kohlenstoff auf, so daß sie nur im Keramiktiegel geschmolzen werden dürfen. Die dunklen Oxide, die sich während des Aufbrennens der Dentalkeramik bei ca. 980˚C bilden, beeinträchtigen durch die Bildung dunkler Säume im Randbereich der Verblendung das ästhetische Erscheinungsbild des Zahnersatzes.

Silberhaltige Palladiumbasislegierungen sind bezüglich ihres Verarbeitungsverhaltens zwischen den hochgoldhaltigen Legierungen und den silberfreien Palladiumbasislegierungen anzusiedeln. Bedingt durch den Silberanteil sind sie besser schmelz – und gießbar, besitzen ein helleres Oxid und zeigen ein gutes Lötverhalten. Gegenüber den silberfreien Palladiumbasislegierungen sind sie außerdem noch preiswerter.

Die typische Zusammensetzung solcher Legierungen ist in der "Übersicht über die Dental – Edelme – tallegierungen und Dental – Nichtedelmetallegierungen in der Bundesrepublik Deutschland" herausgegeben vom Forschungsinstitut für die zahnärztliche Versorgung (FZV), Stand 1. Juli 1986, Seite 31/32, zu finden. Neben Palladium und Silber enthalten diese Legierungen vorwiegend Zinn, Indium und Zink, vereinzelt auch Kupfer oder Gallium als weitere Legierungselemente.

Der Nachteil dieser Legierungen ist, daß sie die Verblendkeramik während des Aufbrennprozesses gelb bzw. gelbgrün verfärben. Ursache hierfür ist das Silber, das durch Diffusion bzw. über die Dampfphase in die Keramik gelangt.

In der DE – PS 25 23 971 werden Palladim – Silber – Legierungen beschrieben, die zu Unterdrückung der Keramikverfärbung 0,1 bis 0,5 % Titan enthalten. Aufgrund der Reaktivität von Titan mit dem Luftsauerstoff bzw. den Tiegelwerkstoffen verarmt die Schmelze relativ schnell an diesem Element, so daß die reduzierende Wirkung auf die Verfärbungsneigung bei Verwendung von Altmaterial (Angußkanäle, Gußtrichter) und bei ungünstig gewählten Schmelzbedingungen verloren geht. Das Titan verursacht außer – dem eine starke Haftung der Einbettmasse an der Oberfläche des gegossenen Objektes, was das Ausbetten und Ausarbeiten schwieriger und zeitaufwendiger macht.

Im US – Patent 4,350,526 werden Palladium – Silber – Legierungen beschrieben, die aufgrund einer Zugabe von 0,1 – 1,0 % Silizium keine verfärbende Wirkung auf Dentalkeramik haben. Silizium ist sowohl in Palladium als auch in Silber unlöslich.

Palladium und Silizium bilden außerdem intermetallische Phasen, so daß eine starke Versprödung der Legierung und Gußbrüchigkeit auftreten können.

Silizium begünstigt – ähnlich wie Titan – die Raktion mit keramischen Werkstoffen, so daß auch bei diesen Legierungen eine starke Haftung der Einbettmasse an dem Gußobjekt auftritt.

In der NL – OS 80 01 820 werden verblendbare Dentallegierungen beschrieben, die 40 bis 60 % Palladium, 24 bis 30 % Silber, 0,05 bis 0,5 % Iridium, 0,05 bis 0,5 % Eisen, Kobalt und/oder Nickel und 0 bis 4 % Gallium enthalten.

Aus der DE – OS 34 38 288 sind verblendbare Dentallegierungen bekannt, die 50 bis 70 % Silber, 15 bis 45 % Palladium, 0,1 bis 2 % Ruthenium, 0,2 bis 10 % Kupfer und/oder Kobalt, 0,2 bis 12 % Indium und/oder Zink, 0,2 bis 5 % Gallium, 0,05 bis 2 % Tantal und/oder Wolfram, 0 bis 5 % Gold und/oder Platin und 0 bis 2 % Zink enthalten können.

In beiden Schriften sind keine Aussagen über die Verfärbungsneigung dieser Legierungen mit Dental‑keramiken gemacht, doch neigen diese Dentallegierungen durch ihren Silbergehalt zu solchen Verfärbun‑gen.

Es war daher Aufgabe der vorliegenden Erfindungen, Palladium‑Silber‑Legierungen zur Herstellung von festsitzendem und herausnehmbaren,

mit Dentalkeramik verblendbaren Zahnersatz zu entwickeln, auf die verfährbungsempfindliche Dentalkera‑miken ohne erkennbare Farbänderungen auf gebrannt und die leicht aus den üblichen Einbettmassen ausgebettet werden können, ohne daß sich die übrigen Eigenschaften der bekannten Palladium‑Silberle‑gierungen signifikant ändern.

Diese Aufgabe wurde erfindungsgemäß gelöst durch die Verwendung von Palladium‑Silber‑Legie‑rungen, bestehend aus 45 bis 80 Gew. % Palladium, 7 bis 50 Gew. % Silber, 0 bis 5 Gew. % Gold, 0 bis 2 Gew. % Platin, 0 bis 5 Gew. % Zinn, 0 bis 5 Gew. % Indium, 0 bis 3 Gew. % Zink, 0 bis 2 Gew. % Kupfer, 0 bis 1 Gew. % Wolfram, Molybdän und/oder Tantal, 0 bis 1 Gew. % Ruthenium, Iridium und/oder Rhenium, 0,5 bis 4 Gew. % Gallium, 1 bis 4 Gew. % Kobalt und 0,1 bis 1,5 Gew. % Germanium, wobei die Summe der Gehalte von Gallium, Kobalt und Germanium zwischen 2 und 7 Gew. % liegen muß.

Besonders vorteilhafte Legierungen enthalten 60 bis 68 Gew. % Palladium, 28 bis 32 Gew. % Silber, 0,1 bis 0,5 Gew. % Ruthenium, Iridium und/oder Rhenium, 1,5 bis 2,5 Gew. % Gallium, 1,5 bis 2,5 Gew. % Kobalt und 1 bis 1,5 Gew. % Germanium.

Palladium‑Silberlegierungen sind ab einem Palladiumgehalt von ca. 25‑30 % mundbeständig. Le‑gierungen mit einem Silbergehalt von mehr als 50 Gew. % besitzen sehr hohe thermische Ausdehnungs‑koeffizienten (> $16,0 \times 10^{-6}$/K), so daß sie mit handelsüblichen Verblendkeramiken nicht mehr kompatibel sind. Bei zu geringen Silbergehalten verhalten sich die Palladium‑Silber‑Legierungen ähnlich wie silber‑freie Palladiumbasislegierungen.

Deshalb wurden Palladium‑Silber‑Legierungen im Bereich von 45‑80 % Palladium und 7‑50 % Silber ausgewählt, die eine hervorragende Mundebeständigkeit zeigen und die Voraussetzung für eine rißfreie Verblendung der Legierung mit Dentalkeramik bieten.

Die Elemente, Zinn, Indium, Zink, Kupfer und Gold dienen der Abstimmung der mechanischen Eigenschaften der Legierungen, wie z.B. Festigkeit, Härte, Gießbarkeit, thermischer Ausdehnungskoeffizient und Schmelzintervall. Ruthenium, Rhenium und/oder Iridium werden in Konzentrationen zwischen 0,1 und 1 % als Kornfeinungszusätze zulegiert.

Palladium‑Silber‑Legierungen, die nur die bisher genannten Legierungselemente enthalten, verfärben die Verblendkeramik jedoch während des Aufbrennprozesses deutlich gelblich‑grün.

Überraschenderweise hat sich nun gezeigt, daß durch den Zusatz von Gallium, Kobalt und Germanium in den erfindungsgemäßen Konzentrationsbereichen die verfärbende Wirkung der oben bezeichneten Legierungen auf die Verblendkeramik deutlich reduziert bzw. beseitigt wird. Ist die Summe der Gehalte der Elemente Gallium, Germanium und Kobalt kleiner als 2 %, so ist die verfärbende Wirkung auf die Keramik noch relativ stark.

Überschreitet diese Summe 7 %, so werden die übrigen Eigenschaften der Legierung negativ beein‑flußt, so daß ein Konzentrationsbereich zwischen 2 und 7 % eingehalten werden muß.

Tabelle 1 zeigt die Zusammensetzung und die Eigenschaften einiger erfindungsgemäßer Legierungen. Sie zeichnen sich durch eine hervorragende Vergießbarkeit, ein helles Oxid und eine problemlose Verblendbarkeit mit den bekannten Dentalkeramiken aus. Die hohe Dehngrenze gepaart mit einer großen Bruchdehnung erlaubt den Einsatz dieser Legierungen auch für den Modellguß.

Die Bestimmung der Farbe der Verblendkeramik auf den Legierungen nach dem Aufbrennen erfolgte mit einem Farbmeßgerät (datacolor, Fa. Light colour systems GmbH, Lichtart D 65, Beobachtungswinkel 10˚) und visuell von mehreren Personen im Vergleich zu einer "Standardprobe". Als Standardlegierung wurde eine goldreduzierte, silberfreie Legierung der Zusammensetzung 52,0 Gew. % Gold, 37,6 Gew. % Palladium, 8 Gew. % Indium, 2,0 Gew. % Gallium und 0,4 Gew. % Iridium, als Keramik die hellste Farbe eines kommerziellen Verblendkeramik‑Sortiments verwendet.

In Tabelle 2 sind die Ergebnisse der Farbmessung an bekannten Vergleichslegierungen und an einigen erfindungsgemäßen Legierungen gemäß Tabelle 1 zusammengestellt (Farbwerte nach CIELab‑System, DIN 6174). Der L‑Wert gibt die Lage auf der Hell‑Dunkel‑Achse an (L = 0 schwarz, L = 100 weiß). Der a‑Wert beschreibt die Lage auf der Grün/Rot‑Achse (negativ = grün, positiv rot), der b‑Wert die Lage auf der Blau‑/Gelb‑Achse (blau negativ, gelb positiv).

In Tabelle 2 sind nicht die absoluten Farbwerte, sondern die Abweichung der Farbwerte für die Keramikverblendung auf den verschiedenen Legierungen von der Standardprobe enthalten ($\Delta$‑Werte) . Die Farbwerte der erfindungsgemäßen Legierungen liegen im Bereich der bekannten silberfreien bzw. hoch‑goldhaltigen Legierungen. Auch bei dem visuellen Betrachten von verblendeten Kronen und Brücken konnte keine Farbabweichung der erfindungsgemäßen Legierungen von der Standardprobe beobachtet werden.

Tabelle 1:

| Legie-rungs-Nr. | Zusammensetzung in Gew. % | | | | | | | | | | | Schmelz-intervalle [°C] | Härte nach Keramik-brand HVS | th.Aus-dehnungs-koeffiz. zwischen Raumtemp. 600°C $10^{-6}K^{-1}$ | 0,2 % Dehn-grenze [MPa] | Bruch-dehnung im Fluß-zustand [%] |
| | Au | Pd | Ag | Ru | Sn | In | Cu | Zn | Ga | Ge | Co | | | | | |
| 1 | – | 63,5 | 30 | 0,5 | 2 | – | – | – | 2 | 1 | 1 | 1270-1170 | 210 | 15,0 | 559 | 23,6 |
| 2 | – | 65,1 | 30 | 0,5 | – | – | – | – | 1 | 1,4 | 2 | 1298-1192 | 230 | n.b. | 552 | 25,7 |
| 3 | – | 62,5 | 30 | 0,5 | 1,0 | 1,0 | – | – | 2 | 1 | 2 | 1249-1155 | 210 | 15,3 | n.b. | n.b. |
| 4 | – | 62,5 | 30 | 0,5 | – | 1 | – | – | 2 | 1 | 3 | 1245-1148 | 240 | 14,9 | n.b. | n.b. |

4

Tabelle 2:

| Legierung | | ΔL | Δa | Δb |
|---|---|---|---|---|
| 1 | Au 52 Pd 37,6 In 8 Ga 2,0 Ir 0,4 (Standard) | - 0,6 | 0,2 | 0,5 |
| 2 | 1 (nach Tabelle 1) | - 1,9 | 0,2 | - 0,1 |
| 3 | " | - 1,6 | 0,2 | 0,2 |
| 4 | Au 77,3 Pd 8,9 Pt 9,8 | - 1,4 | 0,5 | 0,3 |
| 5 | Au 53,2 Pd 35,1 Sn 6,6 Co 2,8 | 0,9 | 0,3 | 1,0 |
| 6 | Pd 79 Ga 9 Cu 9,5 | - 3,1 | 0,1 | - 0,7 |
| 7 | Pd 79,7 Sn 6,5 Ga6 Cu 5 | - 1,7 | 0,5 | 0,3 |
| 8 | Pd 76,5 Cu 11,6 Ga 7,2 | 0,7 | 0,2 | 3,1 |
| 9 | Pd 57,8 Ag 30 Sn 6 In 4 | | | |

(bei den Vergleichslegierungen 4 bis 9 sind nur die Bestandteile angegeben, die über 2 Gew. % liegen)

## Patentansprüche

1. Verwendung von Palladium – Silber – Legierungen, bestehend aus 45 bis 80 Gew. % Palladium, 7 bis 50 Gew. % Silber, 0 bis 5 Gew. % Gold, 0 bis 2 Gew. % Platin, 0 bis 5 Gew. % Zinn, 0 bis 5 Gew. % Indium, 0 bis 3 Gew. % Zink, 0 bis 2 Gew. % Kupfer, 0 bis 1 Gew. % Wolfram, Molybdän und/oder Tantal, 0 bis 1 Gew. % Ruthenium, Iridium und/oder Rhenium, 0,5 bis 4 Gew. % Gallium, 1 bis 4 Gew.

% Kobalt und 0,1 bis 1,5 Gew. % Germanium, wobei die Summe der Gehalte von Gallium, Kobalt und Germanium zwischen 2 und 7 Gew. % liegen muß, zur Herstellung von festsitzendem und herausnehmbaren, mit Dentalkeramik verblendbaren Zahnersatz.

2. Verwendung von Palladium – Silber – Legierungen nach Anspruch 1, bestehend aus 60 bis 68 Gew. % Palladium, 28 bis 32 Gew. % Silber, 0,1 bis 0,5 Gew. % Ruthenium, Iridium und/oder Rhenium, 1,5 bis 2,5 Gew. % Gallium, 1,5 bis 2,5 Gew. % Kobalt und 1 bis 11,5 Gew. % Germanium.

**Claims**

1. Use of palladium – silver alloys, comprising 45 to 80% by weight of palladium, 7 to 50% by weight of silver, 0 to 5% by weight of gold, 0 to 2% by weight of platinum, 0 to 5% by weight of tin, 0 to 5% by weight of indium, 0 to 3% by weight of zinc, 0 to 2% by weight of copper, 0 to 1% by weight of tungsten, molybdenum and/or tantalum, 0 to 1% by weight of ruthenium, iridium and/or rhenium, 0.5 to 4% by weight of gallium, 1 to 4% by weight of cobalt and 0.1 to 1.5% by weight of germanium, where the sum of the contents of gallium, cobalt and germanium must be between 2 and 7% by weight, for the production of fixed and removable false teeth which can be faced with dental ceramic.

2. Use of palladium – silver alloys according to Claim 1, comprising 60 to 68% by weight of palladium, 28 to 32% by weight of silver, 0.1 to 0.5% by weight of ruthenium, iridium and/or rhenium, 1.5 to 2.5% by weight of gallium, 1.5 to 2.5% by weight of cobalt and 1 to 1.5% by weight of germanium.

**Revendications**

1. Utilisation d'alliages palladium – argent constitués de 45 à 80 % en poids de palladium, 7 à 50 % en poids d'argent, 0 à 5 % en poids d'or, 0 à 2 % en poids de platine, 0 à 5 % en poids d'étain, 0 à 5 % en poids d'indium, 0 à 3 % en poids de zinc, 0 à 2 % en poids de cuivre, 0 à 1 % en poids de tungstène, molybdène et/ou tantale, 0 à 1 % en poids de ruthénium, iridium et/ou rhénium, 0,5 à 4 % en poids de gallium, 1 à 4 % en poids de cobalt et 0,1 à 1,5 % en poids de germanium, la somme des teneurs en gallium, cobalt et germanium devant se trouver comprise entre 2 et 7 % en poids, pour préparer des prothèses dentaires fixes et amovibles doublables avec de la céramique dentaire.

2. Utilisation d'alliage palladium – argent selon la revendication 1, constitué de 60 à 68 % en poids de palladium, 28 à 32 % en poids d'argent, 0,1 à 0,5 % en poids de ruthénium, iridium et/ou rhénium, 1,5 à 2,5 % en poids de gallium, 1,5 à 2,5 % en poids de cobalt et 1 à 11,5 % en poids de germanium.